# EUROPEAN PATENT APPLICATION

(11) **EP 2 305 249 A1**
(43) Veröffentlichungstag der Anmeldung: **06.04.2011**
(21) Anmeldenummer: 09766910.5
(22) Anmeldetag: 15.01.2009
(51) Int. Cl.: A61K 31/4152, A61P 43/00

(54) **MEMBRAN-STABILISIERENDES MITTEL**

(30) Priorität: 17.06.2008 RU 2008123590
(71) Anmelder: Hudoley, Vladimir Nikolaevich, Tomskaya obl. 634028 (RU)
(72) Erfinder: Hudoley, Vladimir Nikolaevich, Tomskaya obl. 634028 (RU)
(74) Vertreter: Jeck, Anton
(86) Internationale Anmeldenummer: PCT/RU2009/000005
(87) Internationale Veröffentlichungsnummer: WO 2009/154505

(57) **Zusammenfassung**

Die Erfindung bezieht sich auf die Medizin und kann in der Pharmakologie als Heilmittel mit unspezifischer membranstabilisierender Wirkung angewendet werden. Gemäß der Erfindung wird Jodantipyrin als Heilmittel mit membranstabilisierender Wirkung vorgeschlagen. Die erkannte Eigenschaft ermöglicht es, dieses Mittel bei toxischen Leberschädigungen, Hepatitis, onkologischen Pathologien (zur komplexmedizinischen Behandlung von Krebskranken mittels Strahlentherapie und Chemotherapie) und bei vielen anderen Pathologien, die mit toxischen Körperschädigungen insgesamt und mit Schädigungen von einzelnen Körpersystemen zusammenhängen, einzusetzen.

## Beschreibung

Die Erfindung betrifft ein Mittel mit membranstabilisierender Wirkung nach dem Oberbegriff des Anspruchs.

Die Erfindung bezieht sich auf die Medizin und kann in der Pharmakologie als Präparat mit unspezifischer membranstabilisierender Wirkung angewendet werden.

Stetiges und allgemeines Wachstum der Morbidität, immer häufigere Fälle von Kombinationen verschiedener Krankheiten bei einem Menschen, Neigung zur langwierigen Therapie und Verchronisierung der Krankheitsbilder und der Abläufe sowie die Entwicklung der Erregerresistenz gegen Arzneimittel können zur Zeit als Nachweise der Abschwächung der Schutzsysteme des menschliches Organismus und vor allem des Immunsystems betrachtet werden. Deswegen ist es ziemlich schwer, die Zunahme der Infektionskrankheitshäufigkeit ohne Korrektion von Immunstörungen anhand der entsprechenden immunotropen Arzneimittel zu bekämpfen. Zurzeit ist es offensichtlich, dass die Behandlung von chronischen, infektiös-entzündlichen Prozessen komplexmedizinisch sein muss. Sie muss Chemotherapeutika enthalten, die auf die Entfernung des Infektionserregers abzielen. Sie muss auch immunomodulierende Mittel enthalten, deren Hauptanwendung die Normalisierung der funktionellen Aktivität des Immunsystems des kranken Menschen ist.

Aus dem Stand der Technik ist das Arzneimittel Jodantipyrin mit immunomodulierender Wirkung bekannt. Es beeinflusst den Körper, indem es die Interferonsynthese durch immunkompetente Zellen stimuliert. Dabei stabilisiert es die biologischen Membranen und hält die Viruseindringung in die Zelle auf.

Jodantipyrin ist dem chemischen Aufbau nach ein einzigartiges Präparat. Das Vorhandensein des heterozyklischen Kerns Pyrazolon und des aromatischen Kerns Phenol bedingt seine immunostimulierenden, entgiftenden und antioxidativen Eigenschaften.

Das Vorhandensein der stabilisierenden Wirkung von Jodantipyrin bei chemischer Beanspruchung der Zelle ist jedoch unbekannt. Die Viruseindringung in die Zelle ist ein biologischer Mechanismus, bei dem die Proteine der Membranzelle mitwirken. Die Fachliteratur und die bekannten Informationsquellen enthalten keine Hinweise auf die Anwendung von Jodantipyrin als Stabilisationsmittel der Zellmembran bei chemischer und physikalischer Einwirkung. Gleichzeitig ist das Prinzip der Einwirkung auf die Membran bei Virus- und bei toxischer Einwirkung grundsätzlich ver-, schieden.

Aus dem Patent RU 2006224 (IPC 5 A61K31/00, A61K31/52, veröffentlicht am 30.01.1994) ist das membranstabilisierende und antioxidative Arzneimittel Rikavitum bekannt. Es enthält als wirksame Mittel in g: Riboxin 0,1, Glutaminsäure 0,1, Ascorbinsäure 0,02, Nikotinamid 0,01, Riboflavin 0,002, Pyridoxin 0,002 und Kaliumchlorid 0,05. Jedoch enthält diese Patentschrift keine Angaben über den Wirkungsmechanismus des Präparats auf die Zellmembran bei negativen physikalisch-chemischen Einwirkungen.

Der nächstkommende Stand der Technik gegenüber der Erfindung ist ein Mittel mit membranstabilisierender Wirkung nach dem Patent TU 2253459 (IPC 7 A61 K31/00, A61 P39/06, veröffentlicht am 10.06.2005). Als membranstabilisierendes Mittel wird Polysorb angewendet. Das Präparat stellt hochgereinigtes, wasseraufnehmendes Siliziumdioxid dar. Die hohe Sorptionswirkung von Polysorb, seine adaptogene, antiallergische, antioxidative Wirkung und der membranstabilisierende Effekt vergrößern gegenseitig die Einwirkung auf den Körper. Es wird ermöglicht, Polysorb als Monotherapie anstelle einiger Präparate anzuwenden.

Jedoch liegt dem Wirkungsmechanismus des genannten Mittels die Körperentgiftung zugrunde. Das heißt, die freien Radikale werden blockiert und aus dem Körper entfernt. Dabei ist die antioxidative Einwirkung einer der Aspekte des Stabilisierungsmechanismus der Zelle, denn es kommt nur die Blockierung der freien Radikale zustande, die eine zerstörende Wirkung auf die Membran aufweisen können. Eine eigentliche Einwirkung auf die Membran liegt jedoch nicht vor.

Es ist Aufgabe der Erfindung, die Auswahl an pharmakologischen Mitteln mit membranstabilisierender Wirkung zu erweitern. Dieses Mittel kann bei toxischen Leberschädigungen, Hepatitis, onkologischen Pathologien (bei der komplexmedizinischen Behandlung der Krebskranken mittels Strahlentherapie und Chemotherapie) und bei vielen anderen Pathologien angewendet werden, die mit den toxischen Körperschädigungen insgesamt und mit den Schädigungen von einzelnen Körpersystemen zusammenhängen.

Das technische Ergebnis bei der Verwendung des angemeldeten Mittels ist die Stabilitätserhöhung der Zellenmembran anhand ihrer Strukturänderung. Das verhindert die schädigende Wirkung von toxischen Agenzien.

Die gestellte Aufgabe wird mittels Anwendung von Jodantipyrin als Mittel mit membranstabilisierender Wirkung gelöst.

Die Anwendung von Jodantipyrin mit neuem Bestimmungszweck, d. h. als Mittel mit membranstabilisierender Wirkung, wurde aufgrund der Erkennung von neuen Eigenschaften dieses Mittels möglich.

Diese Eigenschaft von Jodantipyrin ist in der Fachliteratur nicht beschrieben.

### Beschreibung des zu untersuchenden Arzneimittels

Jodantipyrin (Jodantipyrinum) - 1-Phenyl-2,3-dimethyl-4-jodpyrazolon ist ein weißes oder fast weißes Kristallpulver, geruchlos oder mit einem schwachen, spezifischen Geruch. Es ist leicht löslich in Chloroform, mäßig löslich in Ethylalkohol 95 % und praktisch unlöslich in Wasser.

Die Untersuchungen wurde an weißen unverwandten ("nichtlinearen") Mäusen mit einem Körpergewicht von 180 - 200 g vorgenommen.

Angaben über die Tiere, die bei der Untersuchung benutzt wurden, sind der Tabelle 1 zu entnehmen.

**Tabelle 1**

| Angaben über die bei der Untersuchung der akuten Giftigkeit benutzten Tiere | | | | |
|---|---|---|---|---|
| Art der Tiere | Alter, Monate | Anzahl | | Körpergewicht [g] |
| | | Männchen | Weibchen | |
| Ratten | 3 | 32 | 32 | 180 - 220 |

Die Verteilung der Tiere und Gruppen und der Übersichtsplan des Experiments sind in Tabelle 2 dargestellt.

| Versuchsgruppen | | Verordnungsschema für Präparate |
|---|---|---|
| Modell von CCI₄-Hepatitis | | |
| 1 | Vergleichsgruppe | Reinwasser im Laufe von 3 Tagen + Olivenöl ab 5. bis zum 8. Tag täglich |
| 2 | CCI₄-Hepatitis | Reinwasser im Laufe von 3 Tagen, CCI₄ + Reinwasser ab 5. bis zum 8. Tag täglich |
| 3 | Jodantipyrinum + CCI₄ | Jodantipyrinum im Laufe von 3 Tagen, CCI₄ + Jodantipyrinum ab 5. bis zum 8. Tag täglich |

| Modell eines Oxidationsvorgangs, induziert durch fert.-Butyl-hydroperoxid (t-ButOOH) | | |
|---|---|---|
| 1 | Vergleichsgruppe | Reinwasser im Laufe von 5 Tagen |
| 2 | Oxidativer Stress t-ButOOH | Reinwasser im Laufe von 5 Tagen + fert.-Butylhydroperoxid einmalig am 5. Tag |
| 3 | Jodantipyrinum + ButOOH | 1- Jodantipyrinum im Laufe von 5 Tagen + fert.-Butylhydroperoxid einmalig am 5. Tag |

Als experimentelle Modelle zur Beurteilung des Einflusses von Jodantipyrin auf biologische Membranen wurden Tiere mit CCI₄ -Hepatitis und oxidativem Stress verwendet. Der oxidative Stress wurde durch intraperitonale Injektion von fert.-Butylhydroperoxid induziert.

Kohlenstofftetrachlorid wird der mikrosomalen Oxidation unter Beteiligung von Zytochrom P-450 ausgesetzt. Dabei werden elektrophile, alkylierende Intermediate und freie Radikale gebildet. Sie sind fähig, die Per-Oxidationslipide (POL) in biologischen Membranen zu induzieren und die Makromoleküle als Ergebnis der kovalenten Bindung mit diesen Molekülen zu modifizieren. Die CCI₄-Hepatitis wurde durch die Mageninneneinführung von Kohlenstofftetrachlorid in einer Dosis von 1,25 ml/kg in Form von 50%iger Öllösung einmalig im Laufe von 4 Tagen verursacht.

Oxidativer Stress wurde bei den Tieren durch die intraperitonale Injektion von fert.-Butyl-hydroperoxid ("Merck", Deutschland) in einer Dosis von 0,1mMol/kg intraperitonal einmalig induziert. Es ist bekannt, dass sich fert.-Butyl-hydroperoxid als lipophile Substanz schnell über den ganzen Körper verbreitet. Es verursacht durch aktive Sauerstoffformen induzierte Oxidationsreaktionen in Membranen. Daher dient es als Modell, anhand dessen die Effekte der Präparate beurteilt werden können, die die Zerstörung und die Änderung der funktionellen Kenndaten der Zellmembrane verhindern.

Der auf die biologischen Membranen abgezielte Effekt wird auch durch eine Zytolyse und einen Austritt der Fermente mit intrazellulärer Lokalisation ins Blut begleitet. Diese Fermente umfassen: Alanin-Aminotransferase (ALT), Aspartat-Aminotransferase (AST) und Laktatdehydrogenase (LDH). Somit können die Aktivierung von POL in der Leber und im Blutserum sowie die Hyperenzymemia als Indikatoren der Membranpermeabilität gelten.

Die Aktivität von Laktatdehydrogenase (LDH), Alanin-Aminotransferase (ALT), Aspartat-Aminotransferase (AST) wurde nach dem kinetischen Verfahren anhand des Testsatzes der Fa. "Biocon" (Deutschland) bestimmt. Die Aktivität der Fermente wurde in Einheiten per Liter ausgedrückt.

Das Inhibieren der Vorgänge der Lipidperoxidation ist einer der Mechanismen, die die Erhaltung von Struktur und Funktion von Membranlipiden sicherstellen. Deshalb zeugt die abnehmende Speicherung der Lipidperoxidations-Produkte im Blutserum und im Gewebe unter Einwirkung des genannten Präparats davon, dass es die biologischen Membranen stabilisieren kann. Als Marker sind die Standbewertung der primären POL-Produkte, nämlich der Lipid-Hydroperoxide, und die Bewertung von einem der sekundären Metabolite, nämlich des Malondialdehyds (MDA), benutzt. Der MDA-Anteil wurde in nMol pro 1ml Blutserum oder pro 1 g Gewebe berechnet.

Jodantipyrin wurde in der Dosis von 37mg/kg verordnet.

### Ergebnisse in Bezuq auf zwei Versuchsmodelle.

### 1. CCI₄-Hepatitis

**Tabelle 3**

| Der Einfluss von Jodantipyrin (JAP) auf die Enzymaktivität, MDA-Konzen-tration, Hydroperoxide von Lipiden (ROOH) im Blutserum, MDA-Gehalt, ROOH in Rattenleber mit CCI₄- Hepatitis, (M±m, Durchschnitt von 8 Beobachtungen). | | | |
|---|---|---|---|
| Kenndaten | Versuchsgruppen | | |
| | Vergleichsgruppe | CCl₄-Hepatitis | JAP+ CCl₄-Hepatitis |
| ALT, (U/I) | 30,38±6,47 | 113,17±8,12* | 81,20±7,66* |
| LDH, (U/I) | 148,1±6,5 | 312,7±26,2* | 242,0±15,6* |
| MDA im Blutserum, nMol/ml | 4,62±0,22 | 8,65±0,42* | 7,14±0,41* |
| Hydroperoxid des Blutserums, µM/I | 9,58±0,86 | 28,43±3,18* | 20,16±0,98* |
| MDA in Leber, nMol/g | 121,6±5,8 | 303,9±44,9* | 216,1±18,5 |
| Hydroperoxid in der Leber, µMol/g | 5052,1±395,4 | 13220,7±1451,4* | 9253,8±1076,2* |

| | | | |
|---|---|---|---|
| Anmerkung: * - p<0,05, für die Gruppen mit CCI₄-Hepatitis und JAP gegenüber Vergleichsgruppe; für die Gruppe JAP+ CCI₄-Hepatitis gegenüber CCI₄-Hepatitis. | | | |

Die Intoxikation durch Kohlenstofftetrachlorid wurde von einer ausgeprägten Hyperenzymemia begleitet: Die Aktivität von Markerenzymen der Zytolyse nahm um das 2- bis 2,5-fache zu. Der Hydroperoxid-Gehalt und der MDA-Gehalt in der Leber der vergifteten Ratten stieg jeweils um das 2,6- und 2,5-fache an. Die Hydroperoxid-Konzentration im Blutserum nahm unter dem Einfluss des toxischen Mittels fast 3-fach und die Konzentration von MDA 1,9-fach zu.

Die Einführung von Jodantipyrin im Hintergrund von CCI₄-Hepatitis beschränkte zuverlässig den Austritt von ALT und LDH ins Blut.

Die Konzentration von MDA und Hydroperoxiden im Blutserum der mit Jodantipyrin behandelten Tiere mit CCI₄-Hepatitis wurde mit niedrigeren Werten erfasst, obwohl sie die Kennwerte der Tiere aus der Vergleichsgruppe nicht erreichte. Ähnliche Ergebnisse sind auch für den Hydroperoxid-Gehalt in Leberhomogenaten kennzeichnend.

Somit zeugen die ermittelten Daten von der Fähigkeit des Jodantipyrins, die POL-Vorgänge zu verhindern und das Zytolyse-Syndrom zu beschränken.

### 2. Modell des Oxidations-Stresses, der durch fert.-Butyl-hydroperoxid induziert wurde

Der Einfluss von Jodantipyrin auf die Lipidperoxidation im Blutserum beim oxidativen Stress

Um die membranstabilisierenden Eigenschaften zu bewerten, wurde das Modell des oxidativen Stresses gewählt, der durch das fert.-Butyl-hydroperoxid verursacht wird. Dieses Modell ermöglicht es, solche Bedingungen im Organismus zu schaffen, die beim oxidativen Stress kennzeichnend sind, wobei der oxidative Stress durch die Wirkung der aktiven Sauerstoffformen bedingt wird.

**Tabelle 4**

| Der Einfluss von Jodantipyrin (IAP) auf den MDA-Gehalt und den Gehalt an Lipid-Hydroperoxid (ROOH) im Blutserum der Ratten beim oxidativen Stress, induziert durch fert.-Butyl-hydroperoxid (t-ButOOH), (M±m, nMol/ml / µMol/ml, durchschnittliche Werte von 8 Beobachtungen) | | | | | | | | |
|---|---|---|---|---|---|---|---|---|
| MDA, nMol/ml | | | | | | | | |
| Vergleichs gruppe (1) | t-ButOOH (2) | % (2-1) | IAP + t-ButOOH (3) | % (3-1) | % (3-2) | Zuverlässigkeit der Unterschiede(2-1) | Zuverlässigkeit der Unterschiede (3-1) | Zuverlässigkeit der Unterschiede (3-2) |
| 4,5 ±0,25 | 7,8±0,67 | 73,3 | 6,2±0,67 | 37,8 | -20,5 | P < 0,05 | P < 0,1 | P = 0,12 |

| ROOH, µMol/ml | | | | | | | | |
|---|---|---|---|---|---|---|---|---|
| 9,0 ±1,08 | 24,7 ±4,89 | 174, 4 | 16,6±2,9 | 84,4 | -32,8 | P < 0,05 | P < 0,05 | P = 0,21 |

Aufgrund der Versuche wurde festgestellt, dass die intraperitonale Injektion von Hydroperoxid eine merkbare Vergrößerung der Produktmenge der Radikal-Oxidation herbeiführt. Das zeugt von der Aktivierung der Oxidationsvorgänge im Körper. Nach der intraperitonalen Verabreichung von fert.-Butyl-hydroperoxid nahm der Gehalt an primären POL-Produkten (Hydroperoxid) und einem der POL-Endprodukte (MDA) im Blutserum der behandelten Tiere zu.

Die Einführung von Jodantipyrin wurde von keinem zuverlässigen POL-Inhibieren begleitet. Jedoch wurden die niedrigeren Absolutmengen der Lipidperoxidations-Produkte erfasst, als es bei den nicht mit dem oxidativen Stress behandelten Tieren der Fall war (Tabelle 4). Somit zeugt die entstehende Änderungstendenz des Hydroperoxid- und MDA-Gehalts im Blutserum der Ratten unter den Aktivierungsbedingungen der Radikalvorgänge von der Fähigkeit des Jodantipyrins, die Aktivierung von POL zu vermindern.

### Zustandsänderung der Lipidperoxidation in der Leber beim oxidativen Stress

**Tabelle 5**

| Der Einfluss von Jodantipyrin (IAP) auf MDA-Gehalt und Lipid-Hydroperoxide-Gehalt (ROOH) in der Leber der Ratten beim oxidativen Stress, induziert durch fert.-Butylhydroperoxid (t-ButOOH), (M±m, nMol/g von Gewebe, durchschnittliche Werte von 8 Beobachtungen) | | | | | | | | |
|---|---|---|---|---|---|---|---|---|
| MDA, nMol/g Gewebe | | | | | | | | |
| Vergleichsgruppe (1) | t-Bu-tOOH (2) | % (2-1) | IAP + t-ButOOH (3) | % (3-1) | % (3-2) | Zuverlässig keit der Un terschiede (2-1) | Zuverlässig keit der Un terschiede (3-1) | Zuverlässigkeit der Unterschiede (3-2) |
| 109,7 ±5,99 | 184,0± 13,9 | 67,7 | 139,3 ±10,08 | 27,0 | -24, 3 | P<0,05 | P = 0,06 | P< 0,05 |

| ROOH, nMol/g Gewebe | | | | | | | | |
|---|---|---|---|---|---|---|---|---|
| 4920,7 ± 553,7 | 9857,9 ±690,7 | 100, 3 | 7268,3 ±346,4 | 47,7 | -26,3 | P< 0,05 | P < 0,05 | P< 0,05 |

Bei den durch intraperitonale Injektion von fert.-Butyl-hydroperoxid behandelten Tieren nahmen der Lipid-Hydroperoxid-Gehalt und der MDA-Gehalt in der Leber zu.

Der POL-Produkt-Anteil war bei den Tieren, die im Laufe von 5 Tagen vor der fert.-Butyl-hydroperoxid-Injektion mit Jodantipyrin behandelt wurden, zuverlässig niedriger (Tabelle 5).

Aus den Daten der Tabelle 5 geht hervor, dass der Gehalt an primären POL-Produkten und den POL-Endprodukten in der Leber bei peroraler Verabreichung von Jodantipyrin abnimmt. Das zeugt von der Fähigkeit, POL in der Leber zu verhindern und die biologischen Membranen der Hepatozyte zu stabilisieren.

### Aktivitätsänderung der zytolytischen Enzyme beim oxidativen Stress

Die POL-Aktivierung im Körper wird bekanntlich von der Permeabilitätsstörung der biologischen Membrane sowie vom Absterben und von der Zerstörung der Zellen begleitet. Als Ergebnis treten Markerenzyme ins Blut aus.

Als Ergebnis wurde im Rahmen dieses Experiments festgestellt, dass die Einführung von fert.-Butyl-hydroperoxid eine wesentliche Aktivitätserhöhung der Markerenzyme im Blutserum verursachte. Bei den mit Jodantipyrin behandelten Tieren war der AST-Gehalt im Blut zuverlässig niedriger. Jedoch erreichte er nicht die Kennwerte der Vergleichsgruppe (Tabelle 6).

**Tabelle 6**

| Der Einfluss von Jodantipyrin (IAP) auf die Enzymaktivität im Blutserum der Ratten beim durch das fert.-Butyl-hydroperoxid (t-ButOOH) bedingten oxidativen Stress (M±m, U/I, durchschnittliche Werte von 8 Beobachtungen) | | | | | | | | |
|---|---|---|---|---|---|---|---|---|
| AST, U/I | | | | | | | | |
| Vergleichsgruppe (1) | t-Bu-tOOH (2) | % (2-1) | IAP + t-ButOOH (3) | % (3-1) | % (3-2) | Zuverlässigkeit der Unterschiede (2-1) | Zuverlässigkeit der Unterschiede (3-1) | Zuverlässigkeit der Unterschiede (3-2) |
| 151,4 ±27,09 | 304,1 ±24,19 | 100,9 | 237,1 ±27,71 | 56,6 | -22,0 | P< 0,05 | P< 0,05 | P< 0,05 |

Aus den Daten in Tabelle 6 geht hervor, dass Jodantipyrin die Aktivität des Markerenzyms AST zuverlässig vermindert.

Bei der intragastrischen Verabreichung an Ratten von CCI₄-Hepatitis weist Jodantipyrin eine mäßige antioxidative Wirkung und die Fähigkeit zur Einschränkung des Zytolyse-Syndroms auf.

Jodantipyrin zeigt bei der intragastrischen Einführung an Ratten eines durch fert.-Butyl-hydroperoxid induzierten oxidativen Stresses die Tendenz zur Mengenabnahme der POL-Produkte, beschränkt den Austritt von AST ins Blut und zeigt eine Tendenz zur Beschränkung der Zellmembranpermeabilität für ALT und LDH.

Somit zeigen die erzielten Ergebnisse von der Fähigkeit des Jodantipyrins, die Zellmembranen unter dem durch Giftstoffe bedingten oxidativen Stress zu stabilisieren.

### Gewerbliche Anwendbarkeit

Das Mittel mit membranstabilisierender Wirkung gemäß der Erfindung kann in der Pharmakologie bei toxischen Leberschädigungen, Hepatitis, onkologischen Pathologien (bei der komplexmedizinischen Behandlung von Krebskranken mittels Strahlentherapie und Chemotherapie) und bei vielen anderen Pathologien, die mit toxischen Körperschädigungen insgesamt und mit Schädigungen von einzelnen Körpersystemen zusammenhängen, angewendet werden.

## Patentansprüche

1. Anwendung von Jodantipyrin als Mittel mit membranstabilisierender Wirkung.
